# EUROPEAN PATENT SPECIFICATION

(11) **EP 0 639 960 B1**
(45) Date of publication and mention of the grant of the patent: **26.08.1998**
(21) Application number: 92914154.7
(22) Date of filing: 11.05.1992
(51) Int. Cl.: A61F 5/00, A61F 5/01

(54) **A BRACE FOR A KNEE JOINT**
KNIEGELENKSORTHESE
BRIDE POUR ARTICULATION DU GENOU

(43) Date of publication of application: 01.03.1995
(73) Proprietor: SINGER, Samuel, Indiana, PA 15701 (US); FRIED, Jeffrey A., Berardston, MA 01337 (US)
(72) Inventor: SINGER, Samuel, Indiana, PA 15701 (US); FRIED, Jeffrey A., Berardston, MA 01337 (US)
(74) Representative: Müller-Boré & Partner Patentanwälte
(86) International application number: US9203903
(87) International publication number: WO9322992

(56) References cited:
- DE-A- 3 905 837
- FR-A- 2 486 388
- FR-A- 2 552 660
- FR-A- 2 581 859
- GB-A- 2 215 213
- US-A- 3 026 869
- US-A- 4 088 130

## Description

### BACKGROUND OF THE INVENTION

### Field of the Invention

This invention relates to an orthopedic support device, specifically a rear strut stabilized, derotational brace which may be used for prophylactic, functional and rehabilitative applications, particularly for a knee joint.

### Background Information

Generally knee braces may be classified according to the function they perform. Prophylactic knee braces are used to reduce the likelihood of injury during activities, particularly sports, in which high loads are placed on the knee joint. Usually, an objective of prophylactic knee braces is to provide some support for the joint without unduly restricting movement, thus reducing the risk of injury to a normal knee joint. Functional knee braces are used to support and stabilize injured joints, and hence, provide the strongest reinforcement for the knee. They often prohibit certain movements. Rehabilitative knee braces are used to support previously injured joints and are used extensively during post operative and rehabilitation periods. Their designs generally lie between prophylactic and functional braces in the amount of support they provide, and the degree of restriction they impose on joint movement is usually adjustable to provide only a specific range of desired motion.

From US-A-3 026 869 there is already known a brace comprising first and second rigid support members. From FR-A-2 581 859 there is known a stiff anterior cuff member secured to a rigid support member of a brace. From GB-A-2 215 213 there are known braces having first and second rigid support members and first and second anchoring means as well as a rigid posterior strut. However, there is no adjustability when the strut is attached to the terminal portions. Furthermore, from US-A-4,088,130 there is known a rigid steel member along the thigh and one along the leg. Finally, from FR-A-2 486 388 there are known rigid members, but these members are not on opposite sides right/left.

Prophylactic supports for the knee joint include wraps of adhesive or elastic tape and reinforced neoprene sleeves. These devices, however, do not provide the support generally required. Prophylactic knee braces have been designed as single hinged vertical members placed laterally on one side of the leg and held by cuffs and straps to the thigh and the leg. Since the anatomy in the knee region is naturally offset, the fixation on one side cannot support the brace effectively. These braces tend to slip, thus exerting undue forces acting against the natural kinematics on the knee joint, and also concentrate forces laterally on the knee, which in some cases, increases the risk of medial collateral ligament or anterior cruciate ligament injury. Several studies have demonstrated the ineffectiveness of such braces in preventing knee injury and in some cases, have demonstrated an increase in foot and ankle injuries as well. This may be due to the slippage of the brace which may cause interference with normal kinematic operation of the knee. This results in fatigue of the thigh and leg muscles, increasing the strain on knee ligaments and the knee joint, thus increasing the risk of injury. In addition, the medial collateral ligament can be preloaded by a lateral brace, increasing the probability of a medial collateral ligament rupture. The bunching up of the support materials in the popliteal space behind the knee, in braces which utilize "fillers" between parts, further contributes to this problem.

Functional knee braces typically have medial and lateral rigid supports for the knee joint. Many of these devices have complicated hinge structures designed to accommodate or the femoral rollback which occurs upon flexion of the knee joint. Some of these braces use rigid molded thigh and leg cuffs to support the hinged lateral and medial support members. Other braces have utilized spiral structures and straps to connect the thigh and leg cuffs. These devices have not proven as effective as desired to protect against a knee injury and to support and stabilize an unstable knee or protect an injured knee. The major disadvantage of these rigid braces is slippage due to the conical shape of the lower extremity. With slippage, even a well designed brace can alter the kinematics of the knee and increase the risk of injury. Altering the normal motion of the knee also causes muscle fatigue, thus, increasing risk of injury. These braces with rigid molded cuffs which capture the knee, prevent the musculature surrounding the knee from effectively absorbing the forces induced around the joint. Existing functional braces have not been proven to effectively control anterior instability at high loads.

Another type of knee brace uses an adjustable rigid or semi-rigid member encircling the knee joint which clamps lateral and medial pads against the knee joint. The proper operation of such braces depends on the exact fit on a specific knee, and therefore must be custom fitted. They are also bulky and generally more expensive. These devices tend to unduly restrict motion of the knee joint. Because of their more complex nature and bulkiness, interference with normal knee operation is higher if the hinge is misaligned.

There remains a need therefore for an improved knee brace for prophylactic, functional and rehabilitative applications.

There is also a need for such an improved knee brace which provides the required support for the knee without undue restriction on the natural movement of the joint.

There is also a need for such an improved knee brace which does not increase the risk of injury.

There is a further need for such an improved knee brace which distributes the load to the fleshy parts of the thigh and leg.

There is yet another need for such an improved knee brace which reduces both shear and torsion forces applied to the knee.

There is still another need for such an improved knee brace which does not slip out of position or bind.

There is an additional need for such an improved knee brace which is light weight and easy to apply and remove.

There is also a need for a knee brace which can be readily and easily adjusted to accommodate for individual knee characteristics and provide the surgeon/physiotherapist with a means for adjusting for abnormal knees for either improved brace fit, optimizing brace function/action for individual patients, or compensating for or controlling specific motions or actions. Adjustments may also be required or desirable during the course of treatment to accommodate for reduction in swelling, changes caused by treatment, or growth as in a child. It would also be desirable to have a single, or only a few basic sizes of the brace, which could be readily adapted to the circumstances, as opposed to the custom modeling and fabrication or the stocking of numerous sizes as required by many other braces.

### SUMMARY OF THE INVENTION

These and other needs are satisfied by the invention according to claim 1. According to one aspect a rigid posterior strut is located in the popliteal space of the knee and to which all other parts of the brace are attached. The popliteal space behind the knee is the ideal location for fixing the reference point for a knee brace because (a) a rigid strut in the popliteal space does not interfere with the natural motion of the knee and/or the natural action of the leg; (b) any external forces acting on the knee through the mechanical strut are least likely to cause injury to the wearer, since the popliteal space is the only part of the knee where there is adequate soft tissue to absorb harmful energy; (c) the flexion and extension action of the knee relocates the brace into its proper position due to the action of the soft tissue in the popliteal region on the strut; and (d) the posterior strut provides a fixed reference point to which other functional parts of the brace may be attached giving design flexibility for prophylactic, functional and rehabilitative applications.

Because the knee brace of this invention has a posterior strut to which vertical supports are connected by hinges, laterally applied forces at the knee are transmitted to the medial side of the brace, thus preventing injury to the medial collateral ligament. Also, medially directed forces are transmitted to the lateral side, bypassing the knee joint.

The embodiment of the knee brace of this invention for prophylactic applications has a lateral inferior rigid support member which attaches to the leg, and a medial superior rigid support member which attaches to the lower thigh. Both of the rigid support members are pivotally connected on terminal portions of the rigid posterior strut. The pivot points are selected to reproduce the natural kinematics of the knee joint, including posterior femoral rollback. In the preferred form of the brace, stiff cuff members, preferably generally triangular in shape, are secured to the rigid support members and extend around the front of the thigh and calf and are pivotally connected to the opposite terminal portion of the posterior strut. The rigid support members are secured to the leg and thigh, respectively, by anchor means, preferably in the form of soft, resilient sleeves which may be held in place by straps secured such as by VELCRO fasteners. Any open spaces between the rigid support members, cuffs, thigh and calf are filled by the resilient sleeves, which, however, do not extend around the knee joint.

The rigid posterior strut transfers forces acting on the knee around critical areas, dissipating some of the forces so transferred into the soft tissue surrounding the popliteal space. The action of the soft tissues on the posterior strut during normal extension/flexion of the knee causes the brace to self-center. The rigid posterior strut also serves as a known fixed anchor point for the elongated hinged support members and the upper and lower cuffs, and may be used to attach any reinforcing, adjusting or motion correcting straps which may be desired for a specific injury. The upper and lower stiff cuffs attached as they are over the soft, resilient sleeves distribute forces acting on the knee through a wide area of the primarily soft tissue in the medial thigh and lateral upper leg areas. They also assist the rigid posterior strut in transmitting torsional forces around the knee joint.

The invention further includes a posterior strut, and a knee brace incorporating the posterior strut, which is adjustable in many degrees of freedom to accommodate for individual joint characteristics and abnormalities, and changes thereof, and to reduce the number of sizes of the brace which must be stocked.

More particularly, terminal members of the strut are connected to the arcuate center member by mounting means adjustably fixing the terminal members to the ends of the arcuate member in selected positions. Such adjustments include axial and rotational positioning of the terminal members relative to the longitudinal axis or the arcuate member. They may further include rotational adjustment of the terminal members about lateral and vertical axis transverse to the longitudinal axis or the arcuate member.

The mounting means also includes attachment means fixing the terminal members in selectable positions. Preferably, the attachment means is a ball and socket connection. With this arrangement, the terminal members may be fixed in any angular position desired relative to the arcuate member and the anterior/posterior size of the strut can be adjusted.

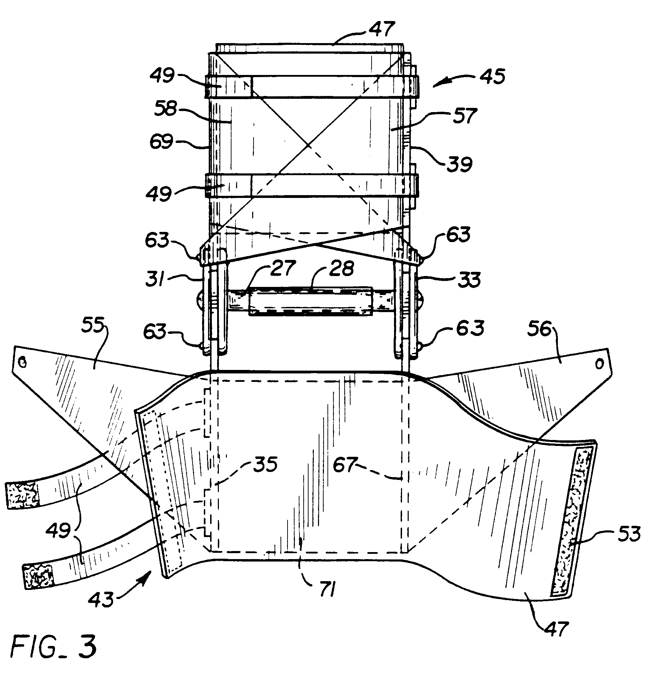

The knee brace incorporating the strut has generally triangular, stiff cuffs secured along one edge to one of the support members hinged to the terminal members and extending either upward along the thigh or downward along the leg. The opposite vertex is pivotably: connected to the terminal member on the opposite side. These cuffs are anterior to the thigh and leg and are bowed so that they can accommodate for small adjustments of the terminal members. For larger adjustments, the cuffs are attached to the elongated support member by a pivot member and fastening means spaced from the pivot member which accommodates for rotation of the cuff about the pivot member to a selected angular position and fixedly secures the cuff to the elongated support member at the selected angular position. Preferably, the fastening means are slots in the cuffs and fasteners extending through the slots and clamping the cuffs in the selected positions. The constructions in figures 3 to 6 do not form part of the invention

### BRIEF DESCRIPTION OF THE DRAWINGS

A full understanding of the invention can be gained from the following description of the preferred embodiments when read in conjunction with the accompanying drawings in which:
Figure 1 is a front elevation view of a knee brace in accordance with one embodiment of the invention shown in use.
Figure 2 is an exploded isometric view of the knee brace of Figure 1.
Figure 3 is a front elevation view of a knee brace not in accordance with the invention.
Figure 4 is a side elevation view of the knee brace of Figure 3.
Figure 5 is a side elevation view of a knee brace shown in Fig. 3 and 4, with the leg in extension.
Figure 6 is a side elevation view similar to Figure 5 showing the leg in flexion.
Figure 7 is a vertical section through a terminal portion of the rear strut which forms a part of the knee brace of the invention.
Figure 8 is a top plan view of a terminal portion of the rear strut.
Figure 9 is an isometric view in enlarged scale of a modified, adjustable posterior strut which forms a part of the knee brace of the invention.

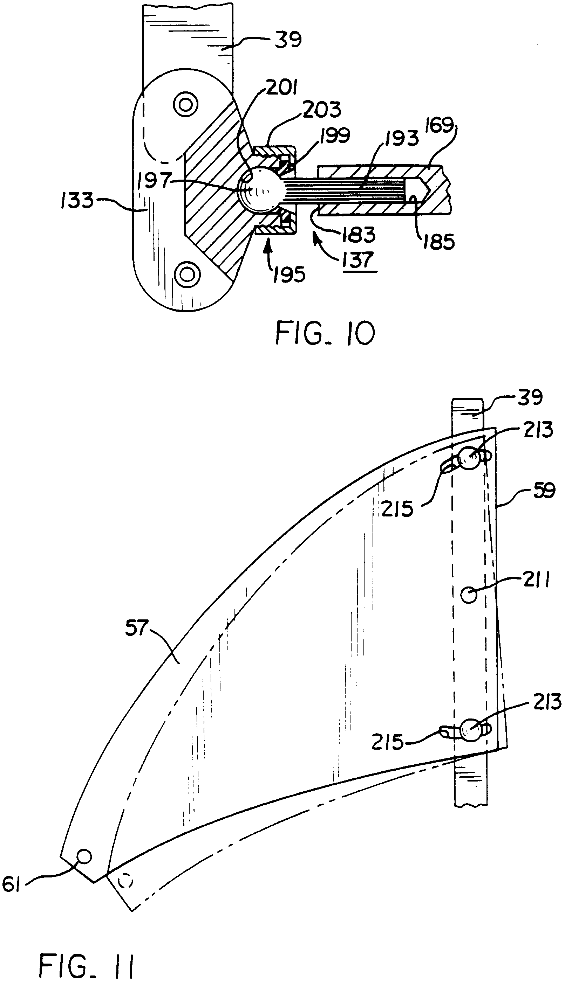

Figure 10 is a fragmentary view, partially in section, of one end of the posterior strut shown in Figure 9.
Figure 11 is a fragmentary side view of a portion of the knee brace of the invention utilizing the adjustable posterior strut of Figures 9 and 10 illustrating a mechanism for adjustment of the cuffs.

### DESCRIPTION OF THE PREFERRED EMBODIMENTS

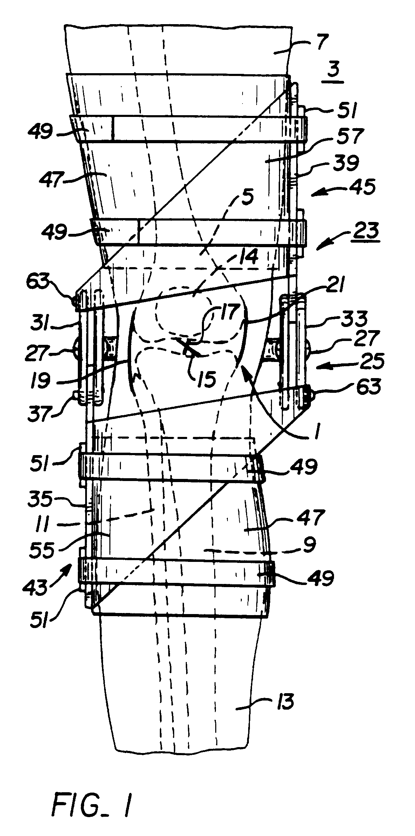

Figure 1 illustrates a knee brace in accordance with the invention in use in supporting and stabilizing a knee joint 1 of a human right lower extremity 3. The knee joint 1 is formed by the enlarged ends of the femur 5, which is the bone of the thigh 7, and the upper end of the tibia 9 which together with the fibula 11 form the bones or the leg 13. The patella (knee cap) 14 articulates with the distal end of the femur 5.

The joint 1 is held together by an arrangement of ligaments including the anterior cruciate ligament 15, the posterior cruciate ligament 17, the lateral collateral ligament 19 and the medial collateral ligament 21. Shear forces and torsional forces applied to the knee joint can result in stretching, and even tearing of these ligaments. A common injury occurs when a lateral blow is applied to the outside of the thigh with the foot planted. This causes the knee joint to buckle inward resulting in tearing of the medial collateral ligament 21, and occasionally the anterior cruciate ligament as well.

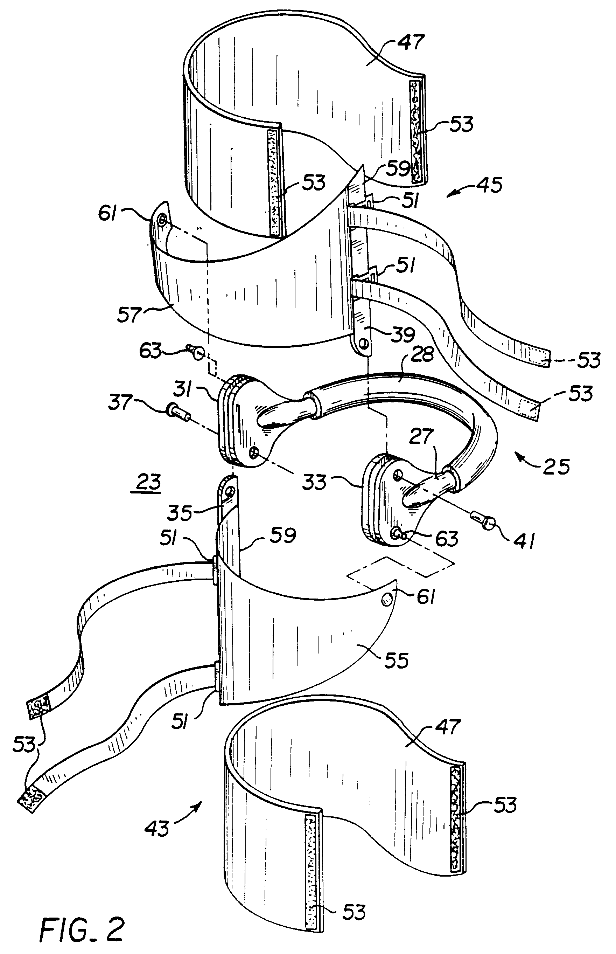

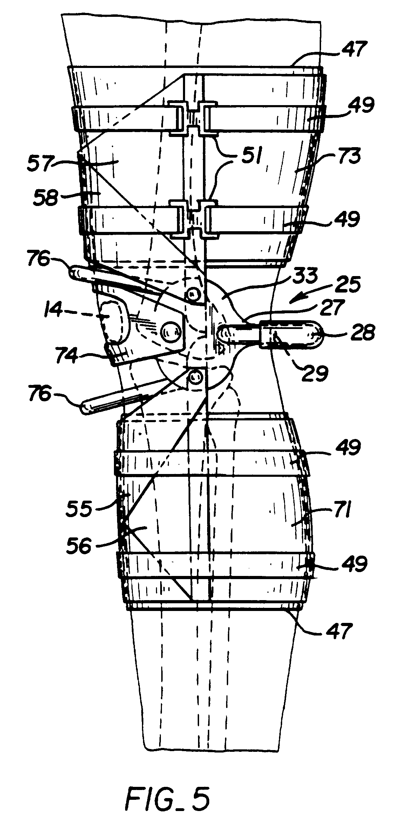

The knee brace 23 shown in Figures 1 and 2 is a prophylactic brace. It includes a rigid posterior strut 25. The strut 25 has an arcuate section 27 which extends behind the knee joint 1 through the popliteal area 29 (see Figure 5) and terminates in enlarged terminal portions 31 and 33 adjacent, but spaced from the lateral and medial sides, respectively, of the joint 1. If desired, the arcuate section 27 can be covered by a soft resilient sleeve 28.

An inferior lateral rigid elongated support member 35 is pivotally connected to the lateral terminal portion 31 of the rigid posterior strut 25 at a first pivot point by a pivot pin 37 and extends down along the lateral side of the leg 13. A superior medial rigid elongated support member 39 is pivotally connected to the terminal portion 33 of the rigid posterior strut 25 at a second pivot point by pivot pin 41, and extends upward medially along the thigh 7.

The support members 33 and 39 are secured to the leg 13 and thigh 7 respectively by anchoring devices 43 and 45. The anchoring devices 43 and 45 each include a sleeve 47 of a non-slip, cushioning material, such as for instance neoprene, and a pair of straps 49 which are threaded through buckles 51 on the support member 35 and 39 and secured by VELCRO fasteners 53. These anchoring devices 43 and 45 firmly secure the support members 35 and 39 to fleshy portions of the leg 13 and thigh 7, respectively, so that forces are transmitted through these support members into the large muscles of the extremity 3.

The prophylactic brace 23 is also provided with a pair of stiff, semirigid anterior cuff members 55 and 57. These cuff members are generally triangular in shape with one edge 59 secured to the associated elongated support member 35 or 39, and with the opposing vertex 61 pivotally connected such as with a snap fastener 63 to a connection point on the terminal portion 31 or 33 of the rigid posterior strut 25 opposite to the terminal portion to which the associated support member 35 or 39 is secured. The pivots formed by the fasteners 63 are laterally aligned with the corresponding pivot points of the elongated members to which the cuffs are attached along the edge 59. The cuff members 55 and 57 are unsnapped and opened for applying the brace 23 to the extremity 3, and then are wrapped around in front of the thigh and leg and snapped in place.

With the prophylactic brace 23 in place, lateral blows to the leg 13 are partially absorbed by the muscles in the leg 13 with the remainder transmitted through the posterior rigid strut 25 to the elongated medial support member 39 which pulls the thigh 7 laterally with the leg and dissipates the transmitted energy into the muscles or the thigh. For a lateral blow to the thigh 7, the force not absorbed by the thigh muscles is transmitted by the elongated support 39, around the knee joint 1 by the rigid posterior strut 25, and through the elongated support member 35 into the fleshy portion of the leg 13. The torsion force generated by rotation of the thigh 7 with the foot planted is transmitted around the knee joint 1 by the rigid posterior strut 25, and through the elongated member 35 into the leg 13. The stiff cuff members 55 and 57 help to balance the rotational forces and to dissipate additional energy into the leg muscles. Anterior and posterior forces applied to the leg 13 or the thigh 7 are similarly transmitted around the knee joint 1 through the rigid posterior strut 25 with the assistance of the stiff cuff members 55 and 57.

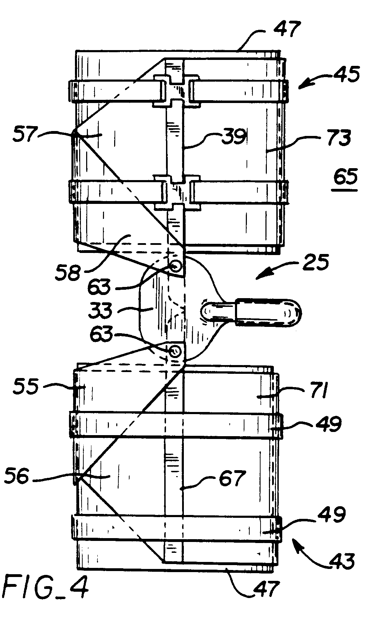

Figures 3 and 4 illustrate a functional brace 65 which is not within the scope of the claimed invention, but serves to illustrate the operation of the brace in accordance with the invention as described in Fig. 1 and 2. The lower portion of brace 65 is shown open in Figure 3 for application to the leg. In this brace, parts corresponding to similar parts in the prophylactic brace 23 of Figures 1 and 2 are identified by the same reference characters. This functional brace 65 also includes an inferior medial rigid elongated support member 67 pivotally connected to the terminal portion 33 of the rigid posterior strut 25 at a pivot point coaxial with the snap fastener 63. The functional brace 65 also includes a superior lateral support member 69 which is pivotally connected to the lateral terminal portion 31 of the strut 25 at a pivot point coaxial with the snap fastener 63, and is secured to the thigh by the straps 49.

The functional brace 65 also includes, in addition to the generally triangularly shaped anterior cuff members 55 and 57, posterior, semicylindrical stiff cuff members 71 and 73 which extend between the respective medial and lateral support members extending along the sides of the leg 13 and thigh 7, respectively.

The brace 65 includes additional stiff, semirigid anterior, generally triangular, cuff member 56, secured to elongated member 67 and connected by a snap fastener 63 to terminal portion 31, and cuff member 58, secured to elongated member 69 and connected by snap fastener 63 to terminal portion 33. These additional cuffs 56 and 58 criss-cross with the cuffs 55 and 57 anterior to the leg and thigh, respectively.

The functional brace 65 with both medial and lateral support members for the thigh and leg and the added cuff members, add additional support and stability to the knee joint.

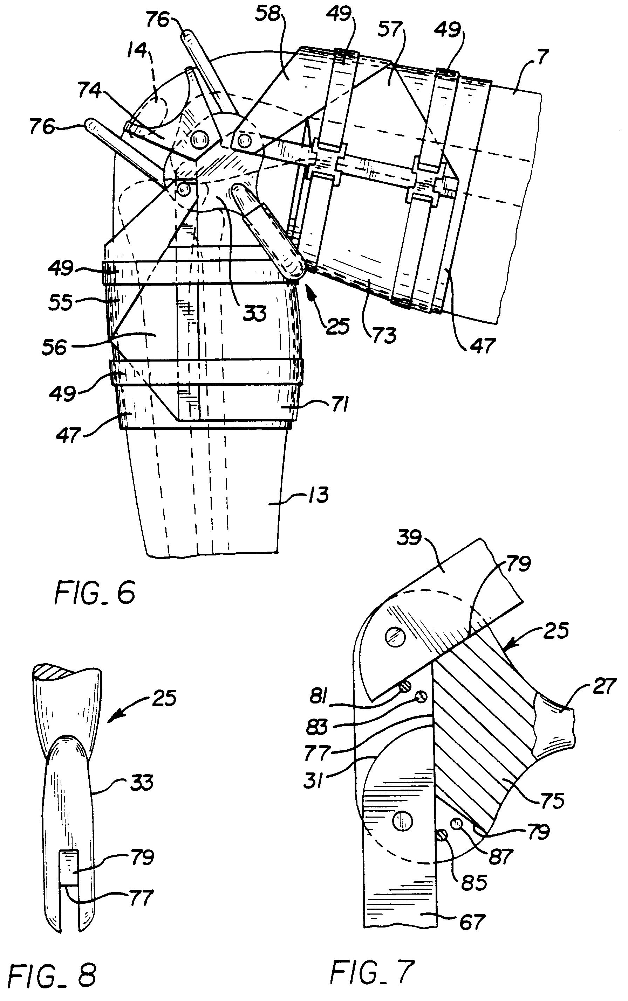

One aspect of knee braces for illustrating the invention is shown in Figures 5 and 6 which are side views of the brace shown in Fig. 3 and 4, not being within the scope of the invention claimed, but useful in explaining the operation of the claimed brace. With the leg extended as shown in Figure 5, the rigid posterior strut 25 extends substantially horizontally, rearward into the popliteal area 29 behind the knee joint 1. When the joint is flexed as shown in Figure 6, the fleshy posterior portions of the leg (the calf) and thigh reposition the rigid posterior strut 25. This repositioning rotates the terminal portions 31 and 33 so that the pivot points at which the inferior and superior support members are attached are rotated to accommodate for the femoral rollback which is illustrated in Figure 6. Thus, in the knee braces in accordance with the invention, the brace is automatically positioned by the natural movement of the leg, thus eliminating the need for complex joint mechanisms which are sometimes ineffective because of slippage of the brace during flexion and extension.

Figures 5 and 6 also illustrates that one or two anterior struts 76 can be secured to the terminal portions 31 and 33 for protecting the knee joint 1 from forces acting frontally or posterior forces forcing the tibia forward. The forces generated by such action are transmitted through the strut 25 to the elongated support members for dissipation in the major muscles in the leg and thigh. The anterior struts 76 form with the posterior strut 25 a rigid support completely surrounding the knee joint and through which forces applied to the limb are transmitted around the knee joint.

The terminal portions 31 and 33 of the rigid posterior strut 25 also serve as reference or attachment points for other devices, such as for instance, a patella stabilizer 74 as shown in Figures 5 and 6.

The hinges formed by the terminal portions 31 and 33 of the rigid posterior strut 25 and the elongated support members can be configured to limit movement of the joint for functional or rehabilitative applications. For instance, as shown in Figure 7, which is a vertical section through the terminal portion 33 of the strut 25, a cam 75 has a vertical edge 77 which is engaged by the pivoting elongated support means such as 39 and 67 to limit extension of the joint, and biased shoulders 79 which restrict rotation of the elongated members to limit flexion of the joint. A pin 81 may be inserted in one of a number of holes 83 to limit extension of the knee joint to less than full extension. Similarly, a pin 85 may be inserted in a selected hole 87 to restrict flexion.

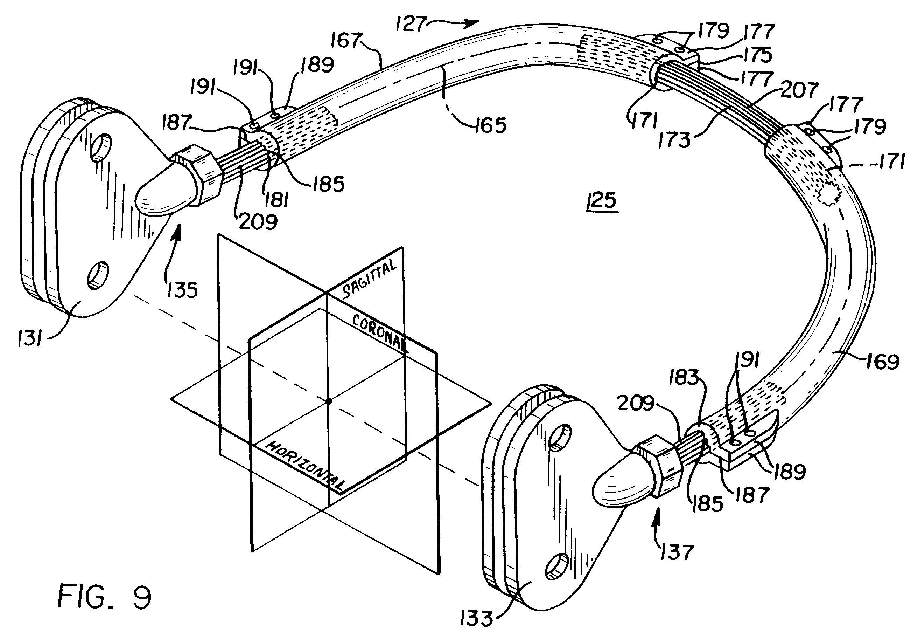

Figure 9 illustrates in detail a modified form 125 of the rigid posterior strut 25 which is adjustable. The strut 125 comprises an arcuate member 127 and terminal member 131 and 133 which are secured to the arcuate member 127 by mounting members 135 and 137 respectively. The arcuate member 127 has a longitudinal axis 165 and is composed of two halves 167 and 169, each of which is substantially a 90° sector or a circle. Confronting ends of the halves 167 and 169 have longitudinal bores 171. An elongated piece in the form of a pin 173 telescopes into the bores 171. Each of the halves 167 and 169 has a slit 175 extending the length of the bore 171. Confronting radial flanges 177 along either side of the slits 175 are drawn together by screws 179 to form clamps which secure the ends of the pin 173 in the bores 171 in a fixed position.

The free ends 181 and 183 of the halves 167 and 169, respectively, have longitudinal bores 185. Slots 187 through the walls or the arcuate members extend a substantial length along the bores 185. Confronting radial flanges 189 on either side of the slots 187 are drawn together by additional screws 191 to form clamps for securing the mounting members 135 and 137 to the arcuate member 127 as will be seen.

The mounting members 135 and 137 each have an elongated member in the form of a stem 193 which telescopes into the bore 185 in one of the halves 167 and 169 of the arcuate member 127 and is secured in a fixed position by the clamps formed by the flanges 189 and screws 191.

The terminal members 131 and 133 are secured to the mounting members 135 and 137, respectively, by adjustable attachments on the mounting members, preferably in the form of ball and socket connectors 195. As shown in Figure 5, these connectors 195 include balls 197 on the ends of the mounting members 135 and 137. Bosses 199 on the rear edges of the terminal members 131 and 133 have rearwardly facing bores with spherical bottoms 201 which define the sockets for the balls 197. Lock nuts 203 which thread onto the outer surface of the bosses 199 clamp an annular ring 205 with a spherical inner surface against the ball 197 to lock the terminal members 131 and 133 in fixed positions relative to the mounting members 135 and 137.

As can be appreciated from Figure 9, the adjustable rigid posterior strut 125 of the invention offers great flexibility in the adjustment of the brace. The adjustable connection between the two halves 167 and 169 of the arcuate member 127 of the posterior strut 125 allows adjustment of the lateral/medial distance between the terminal members 131 and 133 by loosening the screws 179, sliding the stem 173 in the bores 171, and then fixing the two halves in this position by tightening the screws 179. Adjustment of the position of the terminal members 131 and 133 relative to each other in the sagittal plane can be made by again loosening the screws 179 and rotating the halves 167 and 169 about the stem 173 to a desired position and then retightening the screws 179. Preferably, the stem 173, and the bores 171 are fluted as at 207 to aid in fixing the angular position of the two halves 167 and 169.

The strut 125 may be adjusted in the anterior/posterior direction through loosening of the screw 191 and sliding the stems 193 of the mounting members 135 and 137 inward or outward in the bores 185 in the ends 181 and 183 of the arcuate halves 167 and 169, and then retightening the screws 191. By loosening the screws 191 and rotating the mounting members 135 and 137 about the longitudinal axis 165 of the arcuate member 127 and then retightening the screws 191, the terminal members 131 and 133 can be rotated in the coronal plane to a desired fixed position relative to the arcuate member 127. Again, the stems 193 and bores 185 can be fluted as at 209 to lock the mounting members 135 and 137 in fixed positions relative to the arcuate members 127 with less torque required on the screws 191.

The ball and socket connectors 195 permit adjustment of the terminal members 131 and 133 in all three planes about the superior/inferior, medial/lateral and anterior/posterior axes relative to the mounting members 135 and 137.

Figure 11 illustrates a mechanism for adjustment of the anterior cuffs 57 which may be necessitated by adjustments, especially large adjustments, to the posterior strut 125. A pin 211 forms a fixed pivot for attaching the cuff member 57 to the elongated support member 39. Fasteners in the form of screws 213 extend through arcuate slot 215, which are generally transverse to the edge 59 of the cuff and spaced above and below the pivot pin 211. The screws 213, which are threaded into the elongated support member 39, are loosened to adjust the angular position of the cuff member 57 to align the vertex 61 with the fastener 63 on terminal member. The need for and the amount of this rotation is determined by the adjustment, if any, made to the posterior strut 125. The screws 213 are then tightened to clamp the cuff member 57 to the elongated support member 39 in the desired fixed angular position. A similar adjustment mechanism is provided for the cuff 55 secured to the other elongated support member 35.

As the adjustable rigid posterior strut 125 serves as the central element to which the other elements or the brace 123 are connected, either directly or indirectly, the various adjustments made to the posterior strut 125 set the position of the other elements. The versatility of the adjustable knee brace in accordance with the invention allows an off the shelf brace to be used for patients with extremities of various sizes and shapes. Only a very few, perhaps two or three, sizes of brace need be stocked, as those stocked parts can be assembled and adjusted to cover a full range of sizes. Furthermore, the versatility of the adjustable brace allows it to be customized for the invariable deviations from the ideal knee joint and for a large range of deformities caused by disease or injury. Furthermore, the brace can be periodically adjusted during use to accommodate for changes such as the growth of a child, or a reduction in swelling.

The following table indicates in general terms exemplary problems or deformities, the conditions which typically give rise to this problem or deformity and the major adjustment to the posterior strut which may be used to compensate for this condition. It will be obvious that in many instances, the major adjustment will give rise to the need for additional adjustments at other connections to maintain the correct kinematics of the knee joint. For instance, if the two halves 167 and 169 of the arcuate member 127 are rotated about the pin 173, it may be necessary to rotate the mounting members 135 and 137 about the stem portions 193 and to adjust the ball and socket connections to maintain the terminal members 131 and 135 generally perpendicular to the axis of rotation of the knee joint.

**TABLE 1**

| COMMON KNEE PROBLEMS AND RELATED CORRECTIONS USING POSTERIOR STRUT BRACE | | |
|---|---|---|
| PROBLEM OR DEFORMITY | COMMON ASSOCIATED CONDITIONS | CORRECTION OF PROBLEM |
| Varus Deformity (bowed-kneed) | Osteoarthritis, Developmental | Tilt Posterior Strut in Coronal Plane (medial high) - rotate 167-169 about 173 |
| Valgus Deformity (knock-kneed) | Rheumatoid Arthritis, Osteoarthritis, Post-Traumatic | Tilt Posterior Strut in Coronal Plane (lateral high) - rotate 167 and 169 about 173 |
| Internal Rotation Deformity (pigeon-toed) | Developmental or Posti-traumatic Posteriolateral Ligamentous Laxity | Adjust Strut in Horizontal Plane Slide 193 in 185 (Elongated Laterally) |
| External Rotation Deformity (ballet dancer) | Developmental or Post-traumatic Posteromedial Ligamentous Laxity | Adjust Strut in Horizontal Plane (extend on one side) - Slide 193 in 185 (Elongated Medially) |
| Flexion Contracture (can't straighten leg) | Osteoarthritis Post-operative, Rheumatoid Arthritis, Post-traumatic | Adjust Strut about Axis of Rotation. Rotate 131 or 133 relative to 127 |
| Extension Deformity | Developmental or Post-traumatic Ligamentous Laxity | Adjust Strut about Axis of Rotation. Rotate 131 or 133 relative to 127 |
| Growing Child | Normal Development of joints | Expand Strut. slide 167 and 169 on 173. Slide 193 out of 185 |
| Right/Left Differences | Various | Individually adjust L/R as required. |
| Swollen Knee | Post-traumatic or Post-operative Phlebitis | Expand Strut Slide 167 and 169 on 173 |
| Atrophied Knee | Post-injury Atrophy | Contract Strut Slide 167 and 169 on 173 |

It can be seen from the above that the ajustable posterior strut 125 provides great versatility to offset the axis of rotation of the brace, as well as individually adjust the lateral to medial distance and offset the vertical axis of action of the affected hinge point to compensate for individual knee characteristics, or to provide the surgeon/physiotherapist a means for adjusting for abnormal knees; for either improved brace fit; optimizing the brace function/action for individual patients; or compensating for, or controlling specific motions or actions.

While specific embodiments of the invention have been described in detail, it will be appreciated by those skilled in the art that various modifications and alternatives to those details could be developed in light of the overall teachings of the disclosure. Accordingly, the particular arrangements disclosed are meant to be illustrative only and not limiting as to the scope of the invention which is to be given the full breadth of the appended claims.

## Claims

1. A brace (23) for a knee joint (1) joining thigh (7) and leg (13) limb members, said brace comprising:
first and second rigid support members (39, 35) extending only on one side of the thigh and one side of the leg respectively, one of the sides being medial and the other side being lateral;
first anchoring means (45) securing said first rigid support member (39) to the thigh (7) along a fleshy portion of said one side of the thigh with a lower end of said first rigid support member adjacent to but spaced from a first side of the knee joint;
second anchoring means (43) securing said second rigid support member (35) to the leg (13) along a fleshy portion of said one side of the leg with an upper end of said second rigid support member adjacent to but spaced from a second side of the knee joint; and
a rigid posterior strut (25) having a first terminal portion (33) pivotally connected at a first pivot point (41) to said lower end of said first rigid support member (39) and inwardly spaced from the first side of the knee joint (1), a second terminal portion (31) pivotally connected at a second pivot point (37) to said upper end of said second rigid support member (35) and outwardly spaced from the second side of the bee joint (1), and a rigid arcuate section (27) extending between said terminal portions through a popliteal space (29) behind the knee joint, said rigid posterior strut (25) being clamped between the thigh and leg to position said first and second pivot points upon flexion of the knee joint.

2. The brace of Claim 1 including:
a first stiff anterior cuff member (57) secured to said first rigid support member (39) and extending around in front of the thigh (7) and pivotally connected to said second terminal portion (31) of said rigid posterior strut (25); and
a second stiff anterior cuff member (55) secured to said second rigid support member (35) and extending around in front of the leg (13) and pivotally connected to said first terminal portion (33) of said rigid posterior strut (25).

3. The knee brace of claim 2 wherein said first and second stiff anterior cuff members (57, 55) are each generally triangular members having a side edge (59) and a vertex (61) opposite the side edge, the side edge (59) of said first stiff cuff member (57) being secured along said first rigid support member (39) and the vertex of the first stiff cuff member pivotally connected to said second terminal portion (31) of said rigid posterior strut (25) at a connection point (63) laterally aligned with the first pivot point (41) on said first terminal portion (33) of said rigid posterior strut (25) and the side edge (59) of said second stiff cuff member (55) being secured along said second rigid support member (31) and the vertex (61) of the second stiff cuff member pivotally connected to said first terminal portion (33) of said rigid posterior strut at a connection point (63) laterally aligned with said second pivot point (37) on the second terminal portion (31) of said rigid posterior strut.

4. The knee brace of any one of Claims 1-3 wherein said rigid posterior strut (125) includes means (137, 135) adjustably fixing said terminal portions (133, 131) of said rigid posterior strut in selected positions relative to said rigid arcuate section (127).

5. The knee brace of Claim 1 wherein said rigid posterior strut (125) is adjustable and comprises:
a rigid arcuate member (127) having first and second ends (183, 181) and a longitudinal axis (165); first and second terminal members (133, 131) having pivot connections for said rigid support members (39, 35); and first and second adjustable mounting members (137, 135) adjustably fixing said first and second terminal members to said first and second ends respectively of said arcuate member in selected positions relative to said arcuate member.

6. The knee brace of Claim 5 wherein said first and second mounting members (137, 135) include elongated members (193) extendable axially with respect to said first and second ends (183, 181) of said arcuate member (127) and rotatable about said longitudinal axis (165) of said arcuate member, and first and second securing means (189, 191) fixedly securing said elongated members in selectable axial and rotational positions relative to said first and second ends respectively of said arcuate member.

7. The knee brace of Claim 6 wherein said first and second mounting members (137, 135) further include first and second adjustable attachment means (195) fixing said first and second terminal members (133, 131) in selectable positions relative to said first and second mounting members.

8. The knee brace of any one of Claims 5-7 wherein said arcuate member (127) comprises first and second halves (169, 167) rotatable about said longitudinal axis (165) to selectable angular positions with respect to one another and are extendable axially along said longitudinal axis to selected axial positions relative to one another, and connecting means (173, 177, 179) fixedly connecting said first and second halves together at a selected angular and a selected axial position.

## Patentansprüche

1. Eine Stütze (23) für ein Kniegelenk (1), das Oberschenkel (7) und Unterschenkelgliedmaßenteile (13) verbindet, wobei die Stütze umfaßt:
erste und zweite steife Stütz- bzw. Tragglieder (39, 35), die sich nur an einer Seite des Oberschenkels bzw. an einer Seite des Unterschenkels erstrecken, wobei eine dieser Seiten medial und die andere Seite lateral ist;
erste Verankerungsmittel (45), die das erste steife Tragglied (39) an dem Oberschenkel (7) entlang einem fleischigen bzw. muskulösen Abschnitt dieser einen Seite des Oberschenkels befestigen bzw. sicherm, wobei ein unteres Ende des ersten steifen Traggliedes benachbart, aber beabstandet von einer ersten Seite des Kniegelenks ist;
zweite Verankerungsmittel (43), die das zweite steife Tragglied (35) an dem Unterschenkel (13) sichern entlang einem fleischigen bzw. muskulösen Abschnitt der einen Seite des Unterschenkels, wobei ein oberes Ende des zweiten steifen Traggliedes benachbart, aber beabstandet von einer zweiten Seite des Kniegelenkes ist; und
eine steife bzw. starre hintere Strebe (25) mit einem ersten End- bzw. Anschlußabschnitt (33), der schwenkbar verbunden ist an einem ersten Schwenk- bzw. Drehpunkt (41) mit dem unteren Ende des ersten steifen Traggliedes (39) und nach innen beabstandet ist von der ersten Seite des Kniegelenkes (1), einen zweiten End- bzw. Anschlußabschnitt (31), der schwenkbar verbunden ist an einem zweiten Schwenk- bzw. Drehpunkt (37) mit dem oberen Ende des zweiten steifen Traggliedes (35) und nach außen beabstandet ist von der zweiten Seite des Kniegelenkes (1) und einen steifen bogenförmigen Abschnitt (27), der sich zwischen den Anschlußabschnitten durch eine Kniekehle (29) hinter dem Kniegelenk erstreckt, wobei die steife hintere Strebe (25) zwischen dem Oberschenkel und dem Unterschenkel geklemmt ist, um die ersten und zweiten Schwenk- bzw. Drehpunkte bei Beugung des Kniegelenks zu positionieren.

2. Die Stütze nach Anspruch 1 umfassend:
ein erstes steifes vorderes Manschettenglied (57), das an dem ersten steifen Tragglied (39) gesichert bzw. befestigt ist und sich vor dem Oberschenkel (7) herumerstreckt und drehbar mit dem zweiten Anschlußabschnitt (31) der steifen hinteren Strebe (25) verbunden ist; und
ein zweites steifes vorderes Manschettenglied (55), das an dem zweiten steifen Tragglied (35) gesichert bzw. befestigt ist und sich vor dem Unterschenkel (13) herumerstreckt und schwenk- bzw. drehbar mit dem ersten Anschlußabschnitt (33) der steifen hinteren Strebe (25) verbunden ist.

3. Kniestütze nach Anspruch 2, bei welcher die ersten und zweiten steifen vorderen Manschettenglieder (57, 55) jeweils im allgemeinen dreieckige Glieder sind mit einer Seitenkante (59) und einem Scheitel bzw. einer Ecke (61) gegenüberliegend der Seitenkante, wobei die Seitenkante (59) des ersten steifen Manschettengliedes (57) entlang dem ersten steifen Tragglied (39) befestigt ist und die Ecke des ersten steifen Manschettengliedes schwenk- bzw. drehbar an dem zweiten Anschlußabschnitt (31) der steifen hinteren Strebe (25) befestigt ist an einem Verbindungspunkt (63), der lateral ausgerichtet ist mit dem ersten Schwenk- bzw. Drehpunkt (41) an dem ersten Anschlußabschnitt (33) der steifen hinteren Strebe (25) und die Seitenkante (59) des zweiten steifen Manschettengliedes (55) entlang dem zweiten steifen Tragglied (31) befestigt ist und die Ecke (61) das zweiten steifen Manschettengliedes schwenk- bzw. drehbar an dem ersten Anschlußabschnitt (33) mit der steifen hinteren Strebe befestigt ist an einem Verbindungspunkt (63), der lateral ausgerichtet ist mit dem zweiten Schwenk- bzw. Drehpunkt (37) an dem zweiten Anschlußabschnitt (31) der steifen hinteren Strebe.

4. Kniestütze nach einem der Ansprüche 1 bis 3, bei welcher die steife hintere Strebe (125) Einrichtungen (137, 135) umfaßt, die die Anschlußabschnitte (133, 131) der steifen hinteren Strebe einstellbar fixieren in ausgewählten Positionen relativ zu dem steifen bogenförmigen Abschnitt (127).

5. Kniestütze nach Anspruch 1, bei welcher die steife hintere Strebe (125) einstellbar ist und aufweist:
ein steifes bogenförmiges Glied (127) mit ersten und zweiten Enden (183, 181) und einer Längsachse (165); erste und zweite Anschlußglieder (133, 131) mit Schwenk- bzw. Drehverbindungen für die steifen Tragglieder (39, 35); und erste und zweite einstellbare Montageglieder (137, 135), die die ersten und zweiten Anschlußglieder einstellbar an den ersten bzw. zweiten Enden des bogenförmigen Gliedes in ausgewählten Positionen relativ zu dem bogenförmigen Glied fixieren.

6. Kniestütze nach Anspruch 5, bei welcher die ersten und zweiten Montageglieder (137, 135) gestreckte Glieder (193) umfassen, die axial verlängerbar sind bezüglich der ersten und zweiten Enden (183, 181) des bogenförmigen Gliedes (127) und schwenk- bzw. drehbar sind bezüglich der Längsachse (165) des bogenförmigen Gliedes und erste und zweite Sicherungs- bzw. Befestigungseinrichtungen (189, 191), welche die gestreckten Glieder in auswählbaren axialen und rotatorischen Positionen relativ zu den ersten bzw. zweiten Enden des bogenförmigen Gliedes fest sichern.

7. Kniestütze nach Anspruch 6, bei welcher die ersten und zweiten Montageglieder (137, 135) weiter erste und zweite einstellbare Anbringungseinrichtungen (195) umfassen, die die ersten und zweiten Anschlußglieder (133, 131) in auswählbaren Positionen relativ zu den ersten und zweiten Montagegliedern fixieren.

8. Kniestütze nach einem der Ansprüche 5 bis 7, bei welcher das bogenförmige Glied (127) erste und zweite Hälften (169 167) aufweist, die um die Längsachse (165) in auswählbaren Winkelpositionen bezüglich einander schwenk- bzw. drehbar sind und entlang der Längsachse in ausgewählten axialen Positionen relativ zueinander axial verlängerbar sind, und Verbindungseinrichtungen (173, 177, 179), die die ersten und zweiten Hälften fest miteinander verbinden in einer ausgewählten Winkel- und einer ausgewählten axialen Position.

## Revendications

1. Bride (23) pour une articulation du genou (1) reliant les membres de structure allongée de cuisse (7) et de jambe (13), ladite bride comprenant :
des premier et second éléments (39, 35) de support rigide s'étendant seulement, respectivement, sur un côté de la cuisse et sur un côté de la jambe, l'un des côtés étant médial, et l'autre côté étant latéral ;
un premier moyen (45) d'ancrage fixant ledit premier élément (39) de support rigide à la cuisse (7) le long d'une partie de chaire dudit un côté de la cuisse, une extrémité inférieure dudit premier élément de support rigide étant adjacente mais écartée d'un premier côté de l'articulation du genou ;
un second moyen (43) d'ancrage fixant ledit second élément (35) de support rigide à la jambe (13) le long d'une partie de chaire dudit un côté de la jambe, une extrémité supérieure dudit second élément de support rigide étant adjacente mais écartée d'un second côté de l'articulation du genou ; et
une traverse comprimée postérieure rigide (25) ayant une première partie terminale (33) reliée de façon pivotante au niveau d'un premier point de pivot (41) à ladite extrémité inférieure dudit premier élément (39) de support rigide et écartée vers l'intérieur du premier côté de l'articulation du genou (1), une seconde partie terminale (31) reliée de façon pivotante au niveau d'un second point de pivot (37) à ladite extrémité supérieure dudit second élément (35) de support rigide et écartée vers l'extérieur du second côté de l'articulation du genou (1), et une section arquée rigide (27) s'étendant entre lesdites parties terminales de part et d'autre de la région poplitée (29) située derrière l'articulation du genou, ladite traverse comprimée postérieure rigide (25) étant fixée entre la cuisse et la jambe pour positionner lesdits premier et second points de pivot lors de la flexion de l'articulation du genou.

2. Bride selon la revendication 1, comprenant :
un premier élément (57) formant manchon antérieur raide fixé audit premier élément (39) de support rigide et s'étendant en entourant l'avant de la cuisse (7) et relié de façon pivotante à ladite seconde partie terminale (31) de ladite traverse comprimée postérieure rigide (25) ; et
un second élément (55) formant manchon antérieur raide fixé audit second élément (35) de support rigide et s'étendant en entourant l'avant de la jambe (13) et relié de façon pivotante à ladite première partie terminale (33) de ladite traverse comprimée postérieure rigide (25).

3. Bride pour genou selon la revendication 2, dans laquelle lesdits premier et second éléments (57, 55) formant manchons antérieurs raides sont chacun des éléments globalement triangulaires ayant un bord latéral (59) et un vertex (61) opposé au bord latéral, le bord latéral (59) dudit premier élément (57) formant manchon raide étant fixé le long dudit premier élément (39) de support rigide et le vertex du premier élément formant manchon raide étant relié de façon pivotante à ladite seconde partie terminale (31) de ladite traverse comprimée postérieure rigide (25) à un point de liaison (63) aligné latéralement avec le premier point de pivot (41) sur ladite première partie terminale (33) de ladite barre comprimée postérieure rigide (25), et ledit bord latéral (59) dudit second élément (55) formant manchon raide étant fixé le long dudit second élément (31) de support rigide et le vertex (61) du second élément formant manchon raide étant relié de façon pivotante à ladite première partie terminale (33) de ladite traverse comprimée postérieure rigide à un point de liaison (63) aligné latéralement avec ledit second point de pivot (37) sur la seconde partie terminale (31) de ladite traverse comprimée postérieure rigide.

4. Bride pour genou selon l'une quelconque des revendications 1 à 3, dans laquelle ladite traverse comprimée postérieure rigide (125) comprend un moyen (137, 135) fixant de manière réglable lesdites parties terminales (133, 131) de ladite traverse comprimée postérieure rigide dans des positions choisies par rapport à ladite section arquée rigide (127).

5. Bride pour genou selon la revendication 1, dans laquelle ladite traverse comprimée postérieure rigide (125) est réglable et comprend :
un élément arqué rigide (127) comportant des première et seconde extrémités (183, 181) et un axe longitudinal (165) ; des premier et second éléments terminaux (133, 131) comportant des liaisons de pivotement avec lesdits éléments (39, 35) de support rigide ; et des premier et second éléments (137, 135) de montage réglables fixant, respectivement, de manière réglable, lesdits premier et second éléments terminaux auxdites première et seconde extrémités dudit élément arqué dans des positions choisies par rapport audit élément arqué.

6. Bride pour genou selon la revendication 5, dans laquelle lesdits premier et second éléments (137, 135) de montage comprennent des éléments allongés (193) pouvant s'étendre axialement par rapport auxdites première et seconde extrémités (183, 181) dudit élément arqué (127) et étant mobiles en rotation autour dudit axe longitudinal (165) dudit élément arqué, et des premier et second moyens (189, 191) de fixation fixant, respectivement, de manière fixe, lesdits éléments allongés dans des positions axiale et en rotation pouvant être choisies par rapport auxdites première et seconde extrémités dudit élément arqué.

7. Bride pour genou selon la revendication 6, dans laquelle lesdits premier et second éléments (137, 135) de montage comprennent en outre des premier et second moyens (195) d'attache réglables fixant lesdits premier et second éléments terminaux (133, 131) dans des positions pouvant être choisies par rapport auxdits premier et second éléments de montage.

8. Bride pour genou selon l'une quelconque des revendications 5 à 7, dans laquelle ledit élément arqué (127) comprend des première et seconde moitiés (169, 167) mobiles en rotation autour dudit axe longitudinal (165) dans des positions angulaires pouvant être choisies l'une par rapport à l'autre et qui peuvent s'étendre axialement le long dudit axe longitudinal dans des positions axiales choisies l'une par rapport à l'autre, et des moyens (173, 177, 179) de liaison reliant, de manière fixe, l'une à l'autre, lesdites première et seconde moitiés à des positions angulaire et axiale choisies.
